# EUROPÄISCHE PATENTSCHRIFT

(11) **EP 2 345 391 B1**
(45) Veröffentlichungstag und Bekanntmachung des Hinweises auf die Patenterteilung: **25.05.2016**
(21) Anmeldenummer: 11150863.6
(22) Anmeldetag: 13.01.2011
(51) Int. Cl.: A61F 2/38, A61F 2/00

(54) **Kniegelenkendoprothese**
Knee prosthetic
Prothèse d'articulation du genou

(30) Priorität: 13.01.2010 DE 102010000067
(43) Veröffentlichungstag der Anmeldung: 20.07.2011
(73) Patentinhaber: AESCULAP AG, 78532 Tuttlingen (DE)
(72) Erfinder: Hagen, Thomas, 78532, Tuttlingen (DE)
(74) Vertreter: Hoeger, Stellrecht & Partner Patentanwälte mbB

(56) Entgegenhaltungen:
- EP-A1- 1 159 938
- EP-A2- 0 519 873
- EP-A2- 1 378 216
- DE-A1- 10 012 060
- DE-U1-202009 012 704
- DE-U1-202010 000 037

## Beschreibung

Die Erfindung betrifft eine Kniegelenkendoprothese mit einem Femurteil, einem Tibiateil und einem zwischen dem Femurteil und dem Tibiateil gelagerten Meniskusteil.
Kniegelenkendoprothesen der eingangs beschriebenen Art kommen insbesondere zum Einsatz, wenn natürliche Kniegelenke aufgrund von Traumata oder dauerhaften Abnutzungen derart geschädigt sind, dass die Lebensqualität der Betroffenen dauerhaft herabgesetzt ist. Beispiele für Kniegelenkendoprothesen der eingangs beschriebenen Art sind beispielsweise aus der EP 0 519 873 A2, der EP 1 378 216 A2, der DE 20 2009 012 704 U1 sowie der DE 100 12 060 A1 bekannt.

Die EP 0 519 873 A2 zeigt die Merkmale des Oberbegriffs des Anspruchs 1.

Bekannte Kniegelenkendoprothesen weisen unterschiedliche Defizite auf, so dass sich die vorliegende Erfindung damit befasst, Kniegelenkendoprothesen der eingangs beschriebenen Art insbesondere hinsichtlich ihrer Funktionalität zu verbessern.
Dies wird bei einer Kniegelenkendoprothese der eingangs beschriebenen Art erfindungsgemäß insbesondere dadurch erreicht, dass das Meniskusteil am Tibiateil um eine medialseitig verlaufende Rotationsachse rotierbar gelagert ist, wobei eine Rotationsführungseinrichtung vorgesehen ist zum Erzwingen einer Rotationsbewegung des Meniskusteils relativ zum Tibiateil um die Rotationsachse infolge einer Schwenkbewegung des Femurteils und des Tibiateils relativ zueinander um eine quer zur Rotationsachse verlaufende Schwenkachse, welche Rotationsführungseinrichtung zusammenwirkende erste und zweite Führungselemente umfasst, die einerseits am Femurteil und andererseits am Tibiateil angeordnet oder ausgebildet sind.

Eine derartige Kniegelenkendoprothese ermöglicht eine außermittige Rotation des Meniskusteils relativ zum Tibiateil, wobei dennoch insgesamt eine verbesserte Stabilisierung erreicht wird durch die vorgesehene Rotationsführungseinrichtung. Die Führung wird insbesondere erreicht durch die zusammenwirkenden ersten und zweiten Führungselemente, die einerseits am Femurteil und andererseits am Tibiateil angeordnet sind. Optional können selbstverständlich auch weitere Führungselemente vorgesehen sein, jedoch ist es auch möglich, ausschließlich zwei Führungselemente in der angegebenen Weise vorzusehen. Die vorgeschlagene Kniegelenkendoprothese gestattet es, infolge einer Beugung des Knies gleichzeitig eine Rotation des Meniskusteils relativ zum Tibiateil um die Rotationsachse zu erzwingen. Auf diese Weise lässt sich mit der vorgeschlagenen Kniegelenkendoprothese näherungsweise eine physiologische Kniekinematik rekonstruieren. Die Rotationsführungseinrichtung ist insbesondere geeignet, die Funktion fehlender Kreuzbänder zu simulieren, wodurch die Femurkomponente auf der Meniskuskomponente bei einer Beugung nach posterior bewegbar und das Gelenk auch in Beugung stabilisierbar wird. Insgesamt lässt sich so eine Kraftleistung der Beinmechanik erhöhen und eine verbesserte Quadrizepsleistung erreichen. Je nach Ausgestaltung kann insbesondere auch die Patella besser geführt werden. Bei einer Implantation einer Kniegelenkendoprothese auftretende Patella-Komplikationen können auf diese Weise reduziert werden. Zudem kann, abhängig von ihrer Ausgestaltung, auch eine größere Beugefähigkeit der Kniegelenkendoprothese erreicht werden.

Günstig ist es, wenn das Femurteil eine mediale und eine laterale Kondyle umfasst, welche eine mediale und eine laterale Kondylenfläche aufweisen, welches Meniskusteils eine mediale und eine laterale Gelenkfläche aufweist, an welchen die medialen und lateralen Kondylenflächen mindestens teilweise anliegen. Durch die Ausbildung der beschriebenen Kondylen und Gelenkflächen kann eine Gleit- und/oder Abrollbewegung je nach Ausgestaltung der Form der Kondylen und Gelenkflächen zwischen dem Femurteil und dem Meniskusteil erreicht werden.

Eine besonders günstige Führung des Femurteils am Meniskusteil kann beispielsweise dadurch erreicht werden, dass die mediale und/oder die laterale Kondylenfläche einen konkav gekrümmten Kondylenflächenbereich und dass die mediale und/oder die laterale Gelenkfläche einen zur medialen und/oder lateralen Kondylenfläche korrespondierenden konvex gekrümmten Gelenkflächenbereich umfassen.

Gemäß einer weiteren Ausführungsform der Erfindung kann vorgesehen sein, dass Krümmungsradien der medialen und/oder lateralen Gelenkflächen größer sind als Krümmungsradien der medialen und/oder lateralen Kondylenflächen. Auf diese Weise ist es möglich, einer Gleitbewegung zwischen den Teilen der Prothese auch eine Abrollbewegung zu überlagern. So kann insbesondere das Femurteil infolge einer Beugung des Knies relativ zum Meniskusteil in posteriorer Richtung bewegt werden, wie dies bei einem natürlichen Kniegelenk der Fall ist.

Vorteilhaft ist es, wenn die Rotationsachse durch ein zwischen dem Tibiateil und dem Meniskusteil ausgebildetes Drehlager definiert wird. So lässt sich ein besonders einfacher und kompakter Aufbau der Kniegelenkendoprothese realisieren.

Besonders einfach wird der Aufbau des Drehlagers, wenn es erste und zweite, zusammenwirkende Lagerelemente umfasst, welche einerseits am Tibiateil und andererseits am Meniskusteil angeordnet oder ausgebildet sind. Die Lagerelemente können mit dem Tibiateil und/oder dem Meniskusteil lösbar verbindbar oder dauerhaft mit diesem verbunden sein. Insbesondere können sie auch einstückig mit den jeweiligen Teilen der Kniegelenkendoprothese ausgebildet sein.

Eine besonders einfache und sichere Führung ermöglicht ein Drehlager, wenn die ersten und zweiten Lagerelemente in Form eines Vorsprungs und einer korrespondierenden Ausnehmung ausgebildet sind. Der Vorsprung kann wahlweise am Tibiateil oder am Meniskusteil ausgebildet sein. Entsprechend kann die Ausnehmung am jeweils anderen Teil ausgebildet sein. Die Ausnehmung kann in Form einer Vertiefung oder auch einer Durchbrechung an einem der beiden Teile der Kniegelenkendoprothese realisiert sein.

Die Rotationsachse kann auf einfache Weise dadurch definiert werden, dass die ersten und zweiten Lagerelemente rotationssymmetrisch bezogen auf die Rotationsachse ausgebildet sind.

Um eine definierte Rotationsbewegung um die Rotationsachse zu erreichen, ist es günstig, wenn die ersten und zweiten Lagerelemente rotationssymmetrisch bezogen auf die Rotationsachse ausgebildete erste und zweite, zusammenwirkende Führungsflächen aufweisen. Auf diese Weise kann insbesondere verhindert werden, dass das Meniskusteil und das Tibiateil relativ zueinander bewegt werden in einer Richtung quer zur Rotationsachse, wenn die ersten und zweiten Lagerelemente miteinander zusammenwirken, also insbesondere miteinander in Eingriff stehen.

Vorzugsweise ist eines der Lagerelemente zylindrisch und das andere Lagerelement hohlzylindrisch geformt. Sie können so insbesondere formschlüssig oder teilweise formschlüssig ineinander eingesetzt werden. Denkbar ist es auch, das zylindrisch geformte Lagerelement nur abschnittsweise zylindrisch auszubilden, also bezogen auf die zylindrische Führungsfläche sich in radialer Richtung erstreckende Ausnehmungen am Lagerelement vorzusehen. Eine solche Ausgestaltung schränkt die Funktion im Zusammenwirken mit einem entsprechend hohlzylindrisch geformten Lagerelement nicht ein. Zur Beschränkung eines Rotationswinkels können die Lagerelemente auch nur über einen bestimmten Winkelbereich zylindrisch beziehungsweise hohlzylindrisch geformt sein, um so Rotationsanschläge auszubilden. Dabei ist vorzugsweise ein vom abschnittsweise hohlzylindrisch geformten Lagerelement vorgegebener Winkelbereich größer als ein vom nur teilweise zylindrisch geformten Lagerelement.

Günstigerweise sind die ersten und zweiten Führungsflächen koaxial zur Rotationsachse ausgebildet. Es können wahlweise auch zwei oder mehr Führungsflächen am jeweiligen Führungselement vorgesehen sein mit unterschiedlichen Krümmungsradien oder Durchmessern, um zusätzlich zu einer Bewegungsbeschränkung in einer Richtung quer zur Rotationsachse auch gleichzeitig axiale Anschläge in einer Richtung parallel zur Rotationsachse zu definieren.

Gemäß einer weiteren Ausführungsform kann vorgesehen sein, dass das Drehlager in Form eines Kugelgelenklagers ausgebildet ist. Ein Kugelgelenklager definiert grundsätzlich eine Vielzahl von Rotationsachsen. Die Einschränkung auf eine entsprechende Rotationsachse kann insbesondere dadurch erreicht werden, dass eine Bewegung des Meniskusteils und des Tibiateils relativ zueinander in einer beziehungsweise auf nur eine Ebene eingeschränkt wird, also eine lediglich zweidimensionale Bewegung von Tibiateil und Meniskusteil relativ zueinander ermöglicht wird.

Ein Kugelgelenklager lässt sich besonders einfach ausbilden, wenn es erste und zweite Kugelgelenkflächen umfasst, wenn eine der Kugelgelenkflächen hohlkuglig und die andere Kugelgelenkfläche kugelig geformt ist und wenn eines der Lagerelemente die eine Kugelgelenkfläche und das andere Lagerelement die andere Kugelgelenkfläche umfasst. Die ersten und zweiten Kugelgelenkflächen können wahlweise am Meniskusteil oder am Tibiateil ausgebildet sein. Es ist keine Beschränkung dahingehend erforderlich, beispielsweise die hohlkugelige Gelenkfläche am Meniskusteil vorzusehen. Sie könnte insbesondere auch am Tibiateil ausgebildet oder vorgesehen sein.

Um eine sichere und definierte Führung von Meniskusteil und Femurteil relativ zueinander um eine vom Drehlager definierte Drehachse sicherzustellen, ist es günstig, wenn die ersten und zweiten Kugelgelenkflächen identische oder im Wesentlichen identische Krümmungsradien aufweisen.

Vorzugsweise ist die Rotationsführungseinrichtung ausgebildet zum Erzwingen einer Abrollbewegung des Femurteils und des Meniskusteils aneinander. Die Rotationsführungseinrichtung kann auf diese Weise eine Bewegung des Femurteils und des Meniskusteils relativ zueinander direkt beeinflussen.

Des Weiteren kann es vorteilhaft sein, wenn die Rotationsführungseinrichtung derart ausgebildet ist, dass sie eine Gleitbewegung des Femurteils und des Meniskusteils relativ zueinander ermöglicht. Insbesondere können so das Femurteil und das Meniskusteil kongruent zueinander ausgebildet werden, um eine verbesserte Führung einer Relativbewegung zwischen Femurteil und Meniskusteil zu erreichen.

Gemäß einer weiteren bevorzugten Ausführungsform der Erfindung kann vorgesehen sein, dass die Rotationsführungseinrichtung derart ausgebildet ist, dass sie eine überlagerte Gleit-/Abrollbewegung des Femurteils und des Meniskusteils relativ zueinander ermöglicht. Auf diese Weise kann infolge einer Beugung des Knies, beispielsweise um eine quer zur Rotationsachse verlaufende Schwenkachse, zusätzlich eine Translationsbewegung des Femurteils relativ zum Meniskusteil in posteriorer Richtung ermöglicht werden. So kann eine Kinematik eines natürlichen Knies noch besser nachgebildet werden.

Vorteilhaft ist es, wenn die ersten und zweiten Führungselemente ausgebildet sind zum Definieren eines Rotationswinkels einer Rotationsbewegung des Meniskusteils und des Tibiateils um die Rotationsachse in Abhängigkeit eines Flexionswinkels zwischen Femurteil und Tibiateil. Mit der Rotationsführungseinrichtung ist es so möglich, in definierter Weise das Meniskusteil relativ zum Tibiateil um die Rotationsachse zu rotieren, und zwar in Abhängigkeit eines Flexions- oder Beugewinkels zwischen Femurteil und Tibiateil. Beispielsweise kann der Flexionswinkel definiert werden durch einen Winkel zwischen Längsachsen der Tibia und des Femurs des Patienten, an denen das Tibiateil und das Femurteil verankert sind.

Vorzugsweise ist die Rotationsführungseinrichtung ausgebildet, um ausschließlich eine Abrollbewegung zwischen der medialen und/oder lateralen Kondylenfläche und der medialen und/oder lateralen Gelenkfläche zu ermöglichen. Auf diese Weise kann das Kniegelenk besonders gut stabilisiert werden, was insbesondere bei Patienten mit fehlenden Kreuzbändern hilfreich ist, um ein Luxationsrisiko zu verringern.

Die Funktionsweise der Rotationsführungseinrichtung lässt sich auf einfache Weise dadurch verbessern, wenn das erste und das zweite Führungselement mindestens teilweise aneinander anliegende erste und zweite Führungsflächen umfassen. Durch das aneinander Abgleiten oder Abrollen der ersten und zweiten Führungselementflächen aneinander kann so infolge einer Beugung der Kniegelenkendoprothese eine gewünschte Bewegung zwischen Tibia und Femur um die Rotationsachse erzwungen werden.

Eine besonders gute Führung lässt sich erreichen, wenn das zweite Führungselement im posterioren Bereich des Femurteils ausgebildet ist. Es kann so insbesondere durch ein erstes Führungselement beeinflusst werden, welches beispielsweise im anterioren, das heißt im vorderen Bereich des Tibiateils angeordnet oder ausgebildet ist. So lassen sich insbesondere einfach und sicher in posteriorer Richtung wirkende Kräfte vom Tibiateil in das Femurteil bei einer Beugebewegung einleiten.

Besonders einfach und kompakt ausbilden lässt sich die Kniegelenkendoprothese, wenn das zweite Führungselement am Femurteil im Bereich zwischen den Kondylen ausgebildet ist. Die Funktionalität der Kondylen wird dadurch nicht oder nur unwesentlich eingeschränkt. Insbesondere muss deren Größe im Vergleich zu herkömmlichen Kniegelenkendoprothesen nicht verringert werden.

Günstigerweise weist die zweite Führungselementfläche mindestens einen in Richtung auf das Tibiateil weisenden konvexen Flächenbereich auf. Ein solcher Flächenbereich kann insbesondere mit einem entsprechenden konkaven Flächenbereich der ersten Führungselementfläche in gewünschter Weise zusammenwirken.

Vorteilhaft ist es, wenn die erste Führungselementfläche mindestens einen in Richtung auf das Femurteil weisenden konkaven Flächenbereich aufweist. In Richtung auf das Femurteil weisen bedeutet insbesondere, dass der konkave Flächenbereich an einem entsprechenden Flächenbereich des Femurteils in Anlage gebracht werden kann, um insbesondere eine Rotationsbewegung des Femurteils relativ zum Tibiateil um die Rotationsachse infolge einer Beugung des Knies zu erzwingen.

Besonders einfach ausbilden lässt sich die Kniegelenkendoprothese, wenn die ersten und zweiten Führungselementflächen in Form von Gleitflächen ausgebildet sind. Dabei sind alle Arten von Gleitflächen möglich.

Zum Erzwingen einer definierten Rotationsbewegung des Femurteils und des Tibiateils relativ zueinander, ist es vorteilhaft, wenn die erste und/oder die zweite Führungselementfläche bezogen auf eine Sagittalebene unsymmetrisch ausgebildet sind.

Kräfte in posteriorer Richtung können auf einfache Weise vom ersten Führungselement in das zweite Führungselement eingeleitet werden, wenn das erste Führungselement im anterioren Bereich des Tibiateils ausgebildet ist.

Besonders einfach und sicher lässt sich das Meniskusteil am Tibiateil lagern, wenn das Tibiateil eine in Richtung auf das Meniskusteil weisende Tibiafläche aufweist.

Besonders einfach herstellen lässt sich das Tibiateil, wenn die Tibiafläche eine Tibiaebene definiert.

Der Aufbau der Kniegelenkendoprothese kann weiter vereinfacht werden, wenn die Rotationsachse senkrecht zur Tibiaebene verläuft.

Für eine optimierte Gleitbewegung des Meniskusteils und des Tibiateils relativ zueinander, ist es günstig, wenn das Meniskusteil eine Unterseite aufweist, welche mindestens einen ebenen Flächenbereich aufweist. Sie kann selbstverständlich auch zwei, drei, vier oder mehr ebene Flächenbereiche aufweisen, die voneinander getrennt oder aber auch miteinander verbunden sind.

Vorzugsweise weist das Meniskusteil zwei voneinander getrennte ebene Flächenbereiche auf.

Günstig ist es, wenn jeder ebene Flächenbereich einem der beiden Gelenkflächen des Meniskusteils zugeordnet ist. Dies kann insbesondere dadurch erreicht werden, dass das Meniskusteil zwei Meniskusteilbereiche aufweist, wobei jeweils ein Meniskusteilbereich eine der beiden Gelenkflächen des Meniskusteils umfasst.

Vorteilhaft ist es, wenn das Meniskusteil einen medialen Meniskusteilbereich und einen lateralen Meniskusteilbereich umfasst und wenn der laterale und der mediale Meniskusteilbereich durch ein Verbindungselement miteinander verbunden sind. Insbesondere können sie starr miteinander verbunden sein, wodurch eine Stabilität der Kniegelenkendoprothese deutlich erhöht wird.

Eine Verbindung der lateralen und medialen Meniskusteilbereiche wird besonders einfach, wenn das Verbindungselement in Form eines Stegs ausgebildet ist. Der Steg selbst kann eine beliebige Querschnittsform aufweisen. Vorzugsweise ist er quaderförmig ausgebildet und weist abgerundete Kanten auf. Es ist jedoch auch denkbar, den Steg im Querschnitt zum Beispiel kreisförmig oder oval auszubilden.

Gemäß einer weiteren bevorzugten Ausführungsform der Erfindung kann vorgesehen sein, dass das Verbindungselement eine an der Tibiafläche mindestens teilweise anliegende Verbindungselementfläche aufweist. Die Verbindungselementfläche kann somit auch einen Teil einer Unterseite des Meniskusteils bilden, insbesondere einen ebenen Flächenbereich, welcher mit weiteren Flächenbereichen des Meniskusteils verbunden ist oder an diese angrenzt. Auf diese Weise kann eine am Tibiateil anliegende Gesamtfläche des Meniskusteils maximiert werden, wodurch eine Stabilität der Kniegelenkendoprothese deutlich verbessert werden kann.

Grundsätzlich wäre es denkbar, das erste Führungselement in die Tibiafläche zu integrieren. Vorteilhafterweise ist das erste Führungselement von der Tibiafläche abstehend ausgebildet. Insbesondere kann es mit dem Tibiateil lösbar verbindbar oder dauerhaft fest verbunden sein. Insbesondere kann es einstückig mit dem Tibiateil ausgebildet sein.

Gemäß einer weiteren bevorzugten Ausführungsform der Erfindung kann eine Sicherungseinrichtung zum Sichern des Meniskusteils am Tibiateil in einer Verbindungsstellung vorgesehen sein, in welcher das Meniskusteil und das Tibiateil um die Rotationsachse rotierbar gelagert sind. Die Sicherungseinrichtung dient also dem Zweck, ein Lösen des Meniskusteil und des Tibiateils voneinander zu verhindern, insbesondere dann, wenn die beiden Teile die Verbindungsstellung einnehmen, in welcher sie in definierter Weise infolge einer Beugung des Knies um die Rotationsachse rotiert werden können.

Ein besonders einfacher Aufbau der Sicherungseinrichtung kann erreicht werden, wenn sie erste und zweite, zusammenwirkende Sicherungselemente umfasst, welche einerseits am Meniskusteil und anderseits am Tibiateil ausgebildet sind.

Günstig ist es, wenn die ersten und zweiten Sicherungselemente quer zur Rotationsachse verlaufende Anschlagflächen aufweisen zum Verhindern einer Bewegung des Meniskusteils und des Tibiateils in der Verbindungsstellung voneinander weg. Die Anschlagflächen verhindern insbesondere, dass das Meniskusteil und das Tibiateil relativ zueinander in einer Richtung parallel zur Rotationsachse bewegbar sind, und zwar optional aufeinander zu und/oder voneinander weg.

Günstig ist es, wenn das erste Sicherungselement einen am Tibiateil angeordneten oder ausgebildeten ersten Rückhaltevorsprung umfasst und wenn das zweite Sicherungselement einen am Meniskusteil angeordneten zweiten Rückhaltevorsprung umfasst. Die ersten und zweiten Rückhaltevorsprünge können insbesondere in der Verbindungsstellung aneinander anliegen. Des Weiteren können die Rückhaltevorsprünge auch die oben beschriebenen Anschlagflächen umfassen oder aufweisen.

Ein besonders kompakter Aufbau der Kniegelenkendoprothese lässt sich insbesondere dadurch erreichen, dass der erste Rückhaltevorsprung an oder in einer Ausnehmung des ersten Führungselements angeordnet oder ausgebildet ist. Die Ausnehmung kann beispielsweise in Form eines Rücksprungs oder einer Nut am ersten Führungselement realisiert sein. Insbesondere kann die Ausnehmung teilweise begrenzt werden durch die Tibiafläche des Tibiateils.

Vorzugsweise umfasst das erste Führungselement den ersten Rückhaltevorsprung. So kann ein besonders kompakter Aufbau der Kniegelenkendoprothese erreicht werden.

Günstigerweise umfasst der erste Rückhaltevorsprung eine erste Anschlagfläche, welche von der Tibiaebene beabstandet ist. Beispielsweise kann so das Verbindungselement zwischen die erste Anschlagfläche und die Tibiafläche ganz oder teilweise eingeführt werden, um eine Bewegung des Meniskusteils und des Tibiateils relativ zueinander in einer Richtung parallel zur Rotationsachse zu verhindern.

Vorzugsweise umfasst oder bildet das Verbindungselement den zweiten Rückhaltevorsprung. Das Verbindungselement kann so insbesondere direkt mit dem ersten Rückhaltevorsprung zusammenwirken, welcher beispielsweise am ersten Führungselement vorgesehen oder ausgebildet ist.

Insbesondere ist es günstig, wenn eine Oberseite des Verbindungselements eine zweite Anschlagfläche der Sicherungseinrichtung umfasst oder bildet. Auf diese Weise kann die Kniegelenkendoprothese beziehungsweise deren Sicherungseinrichtung besonders kompakt ausgebildet werden.

Vorzugsweise ist die erste Führungselementfläche bezogen auf die erste Anschlagfläche in posteriorer Richtung versetzt angeordnet oder ausgebildet. Auf diese Weise kann insbesondere erreicht werden, die Funktion der Sicherungseinrichtung und der Rotationsführungseinrichtung räumlich voneinander zu trennen.

Gemäß einer bevorzugten Ausführungsform kann vorgesehen sein, dass das Meniskusteil und das Tibiateil von der Verbindungsstellung in eine Montagestellung, in welcher die ersten und zweiten Anschlagflächen außer Eingriff stehen, bringbar sind durch Rotieren um die Rotationsachse um einen Lösewinkel. Dies kann insbesondere dadurch erreicht werden, dass das Meniskusteil und das Tibiateil relativ zueinander um die Rotationsachse so weit verdreht werden, dass die Anschlagflächen der Sicherungseinrichtung nicht mehr miteinander in Eingriff stehen beziehungsweise zusammenwirken können.

Vorteilhaft ist es, wenn die ersten und zweiten Anschlagflächen in der Verbindungsstellung einen durch mindestens teilweise Überlappung senkrechter Projektionen derselben auf die Tibiafläche definierten Flächenabschnitt definieren. Mit anderen Worten können die ersten und zweiten Anschlagflächen in der Verbindungsstellung direkt miteinander in Anlage gebracht werden, in der Montagestellung dagegen nicht.

Vorteilhaft ist es ferner, wenn die Kniegelenkendoprothese einen Rotationsbegrenzungsanschlag zum Begrenzen einer Bewegung der lateralen Gelenkfläche in anteriorer Richtung umfasst. Insbesondere kann der Rotationsbegrenzungsanschlag so ausgebildet sein, dass er eine Bewegung des Meniskusteils relativ zum Tibiateil um die Rotationsachse derart einschränkt, dass eine Bewegung der lateralen Gelenkfläche in anteriorer Richtung eingeschränkt ist. Vorzugsweise bildet oder umfasst das erste Führungselement den Rotationsbegrenzungsanschlag. So lässt sich die Kniegelenkendoprothese noch kompakter ausbilden.

Vorteilhaft ist es, wenn das Femurteil und/oder das Meniskusteil und/oder das Tibiateil einstückig ausgebildet sind. Sie können wahlweise oder alle einstückig ausgebildet sein.

Insbesondere vorteilhaft ist es, wenn das Femurteil und/oder das Tibiateil in Form modularer Prothesenteile ausgebildet sind. Darunter ist insbesondere zu verstehen, dass das Femurteil und/oder das Tibiateil jeweils einen Schaft aufweisen können, die in entsprechend vorbereitete Kavitäten an Femur oder Tibia des Patienten eingesetzt und darin fixiert werden können, beispielsweise mittels Schrauben oder Knochenzement. Die Schäfte können wahlweise ein oder mehrteilig ausgebildet sein und wahlweise lösbar verbindbar oder zumindest teilweise einstückig dem Femurteil beziehungsweise dem Tibiateil ausgebildet sein. Eine modulare Ausgestaltung von Femurteil und Tibiateil hat den Vorteil, dass diese individuell an die Physiologie des Patienten angepasst werden können.

Vorzugsweise sind das Femurteil und das Meniskusteil aus unterschiedlichen Materialien hergestellt. Das Femurteil, ebenso wie das Tibiateil, sind vorzugsweise aus einem Implantatstahl gebildet oder aus einem anderen, körperverträglichen Metall, beispielsweise Titan. Das Meniskusteil wird vorzugsweise aus einem abriebfesten Kunststoff gebildet, beispielsweise Polyethylen oder Polyethylen mit hoher Dichte und hohem Molekulargewicht.

Die nachfolgende Beschreibung bevorzugter Ausführungsformen dient im Zusammenhang mit den Zeichnungen der näheren Erläuterung. Es zeigen:
- Figur 1:: eine anteriore Seitenansicht eines Ausführungsbeispiels einer Kniegelenkendoprothese;
- Figur 2:: eine perspektivische Ansicht des Ausführungsbeispiels aus Figur 1 in gestreckter Stellung;
- Figur 3:: eine Explosionsdarstellung des Ausführungsbeispiels aus Figur 2;
- Figur 4:: eine Ansicht des Ausführungsbeispiels analog Figur 2 in einer Beugestellung; und

- Figur 5:: eine Draufsicht auf das Ausführungsbeispiel in der in Figur 4 dargestellten Beugestellung.

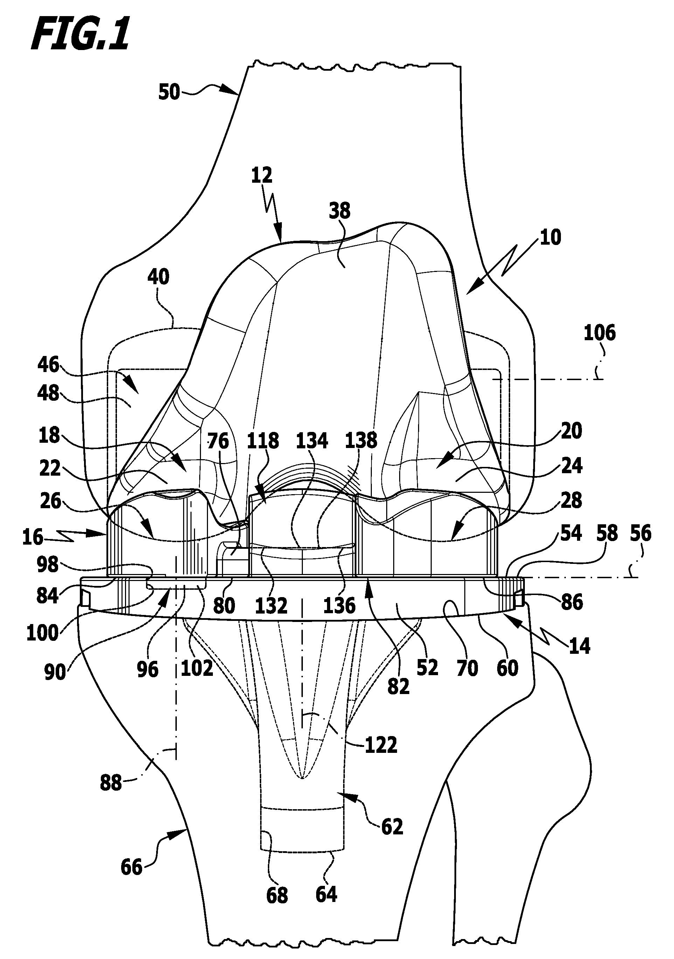

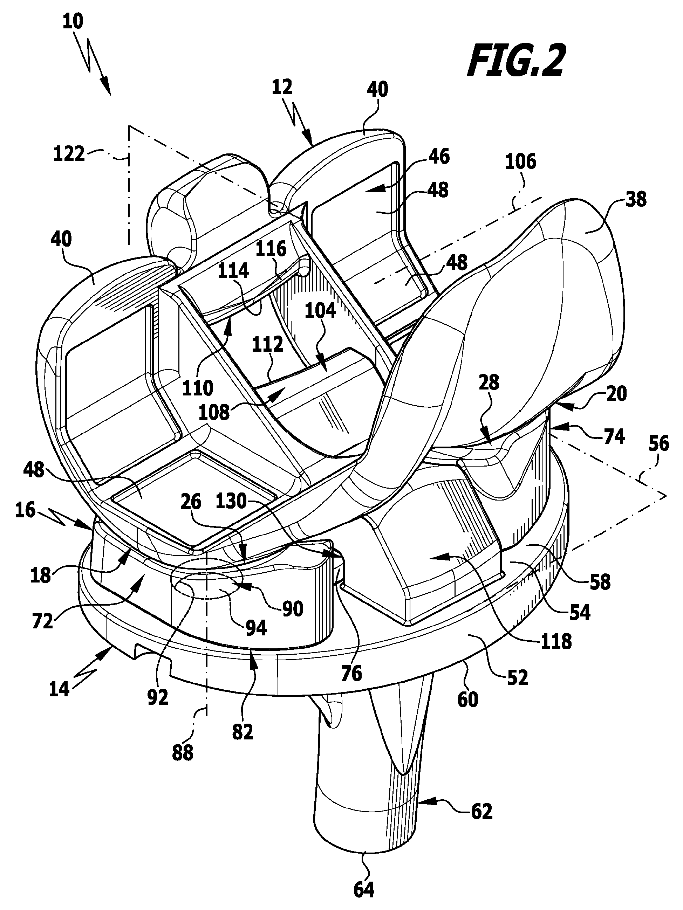

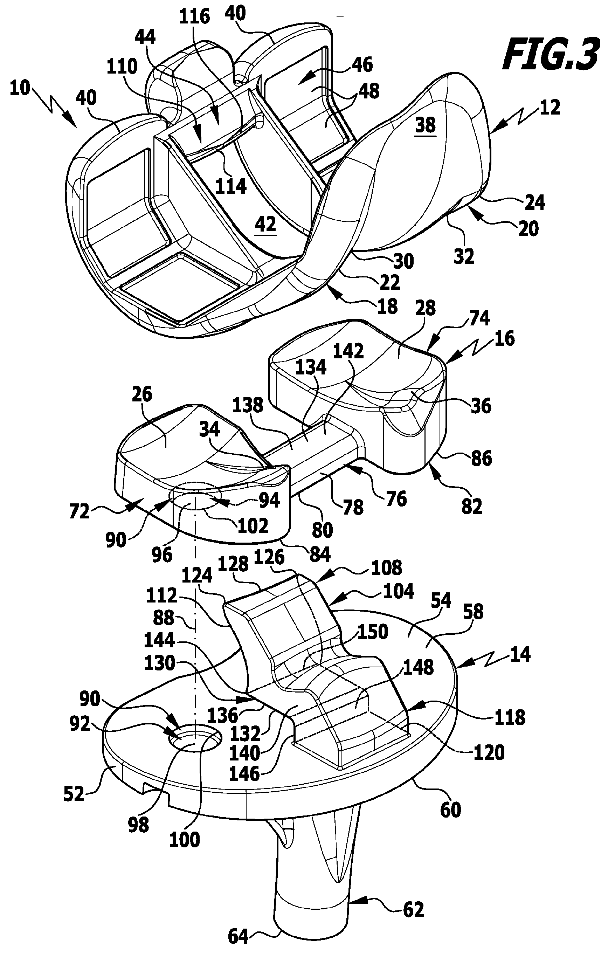

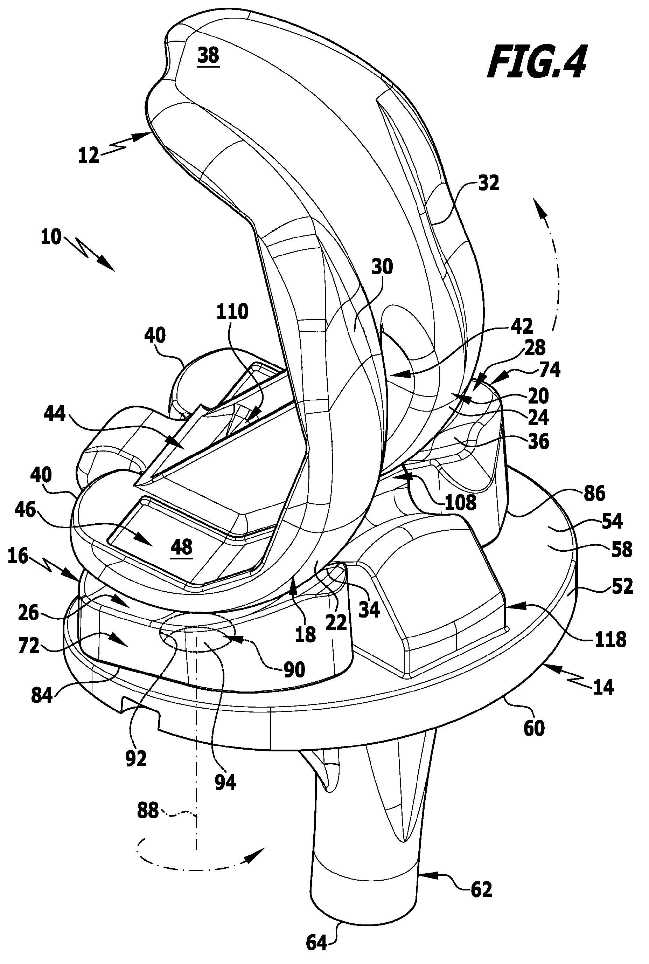

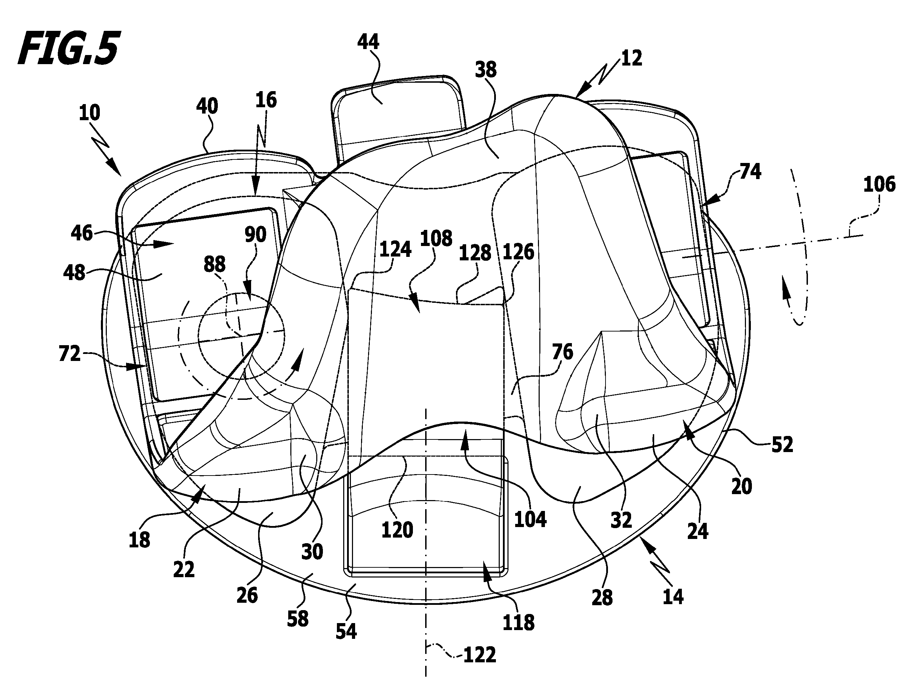

In den Figuren 1 bis 5 ist ein erstes Ausführungsbeispiel einer insgesamt mit dem Bezugszeichen 10 versehenen Kniegelenkendoprothese schematisch dargestellt, welche ein Femurteil 12, ein Tibiateil 14 und ein zwischen dem Femurteil 12 und dem Tibiateil 14 beweglich gelagertes Meniskusteil 16 umfasst.

Das Femurteil 12 umfasst eine mediale Kondyle 18 und eine laterale Kondyle 20, welche eine mediale Kondylenfläche 22 beziehungsweise eine laterale Kondylenfläche 24 aufweisen. Die medialen und lateralen Kondylenflächen 22, 24 des Meniskusteils 16 sind vom Femurteil 12 weg weisend im Wesentlichen konvex gekrümmt und zum Abgleiten beziehungsweise Abrollen an medialen und lateralen Gelenkflächen 26, 28 des Meniskusteils 16 ausgebildet, an welchen sie mindestens teilweise anliegen.

Für eine verbesserte Führung einer Relativbewegung des Femurteils 12 und des Meniskusteils 16 aneinander weisen die mediale und die laterale Kondylenflächen 22, 24 vorzugsweise jeweils einen konkav gekrümmten Kondylenflächenbereich 30, 32 auf, welche im Wesentlichen korrespondierend zum Zusammenwirken mit konvex gekrümmten medialen und lateralen Kondylenflächenbereichen 34, 36 der ansonsten im Wesentlichen vom Meniskusteil 16 weg weisend konkav gekrümmten medialen und lateralen Gelenkflächen 26, 28 ausgebildet sind. Krümmungsradien der medialen und/oder lateralen Gelenkflächen 26, 28 sind vorzugsweise größer als Krümmungsradien der medialen und/oder lateralen Kondylenflächen 22, 24 ausgebildet, so dass nicht nur eine Gleitbewegung zwischen dem Femurteil 12 und dem Meniskusteil 16 realisierbar ist, sondern gleichzeitig auch eine Abrollbewegung, die der Gleitbewegung insbesondere überlagert sein kann.

Die Kondylen 18 und 20 sind an ihrem vorderen oder anterioren Ende 38 miteinander verbunden. Zwischen ihrem anterioren Ende 38 und einem posterioren Ende 40 ist ein Spalt oder Zwischenraum 42 zwischen den beiden voneinander beabstandeten Kondylen 18 und 20 ausgebildet. Im Bereich ihrer posterioren Enden 40 sind die Kondylen 18 und 20 über ein Verbindungselement 44 miteinander fest verbunden.

Die Kondylenflächen 22 und 24 bilden im Wesentlichen eine Vorder- oder Außenseite des Femurteils 12. In entgegengesetzter Richtung weisend, das heißt auf einer Rückseite oder Innenseite der Kondylen 18 und 20 sind jeweils Anlageflächen 46 ausgebildet, welche insbesondere mehrere relativ zueinander geneigte, ebene Anlagenflächenbereiche 48 aufweisen können. Zum Fixieren des Femurteils 12 an einem Femur 50 eines Patienten wird der Femur 50 entsprechend der Rückseite des Femurteils 12 präpariert, das heißt es werden in den Figuren nicht näher dargestellte Anlageflächen am Femur 50 präpariert, die zu den Anlagenflächen 46 korrespondieren, um das Femurteil 12 beispielsweise mit Knochenzement am Femur 50 festzulegen. Optional können auch nicht dargestellte Knochenschrauben zum Einsatz kommen, um das Femurteil 12 alternativ oder zusätzlich am Femur 50 zu sichern.

In den Figuren ebenfalls nicht dargestellt sind alternative Ausführungsformen von Femurteilen 12, die zusätzlich einen oder mehrere vom Femurteil 12 abstehende Schaftabschnitte aufweisen, die in entsprechende Ausnehmungen, die zuvor am Femur 50 präpariert wurden, eingesetzt und mittels Knochenzement und/oder Befestigungselementen, wie beispielsweise Knochenschrauben, festgelegt werden können. Derartige Schäfte oder Schaftabschnitte können insbesondere modular ausgebildet sein, um ihre Länge entsprechend optimiert an eine Physiologie des Patienten anpassen zu können.

Das Tibiateil 14 umfasst eine Platte 52, welche eine in Richtung auf das Meniskusteil 16 weisende Tibiafläche 54 aufweist, die eine Tibiaebene 56 definiert. Somit ist die Tibiafläche 54 eben. Die Tibiafläche 54 bildet eine Oberseite 58 der Platte 52, die in einer Draufsicht im Wesentlichen nierenförmig geformt ist. Von einer Unterseite 60 der Platte 52 ist ein Schaft oder Schaftabschnitt 62 abstehend angeordnet oder ausgebildet. Ein distales Ende 64 desselben kann optional mit Verlängerungselementen verbunden werden, die in eine entsprechend vorbereitete Kavität 68 einer Tibia 66 eingesetzt werden können. Zum Anlegen der Platte 52 wird die Tibia 66 teilreseziert und eine ebene Anlagefläche 70 präpariert, an welcher die Unterseite 60 im Wesentlichen großflächig anliegt.

Das Meniskusteil 16 umfasst einen medialen Meniskusteilbereich 72 und einen lateralen Meniskusteilbereich 74. Der laterale und der mediale Meniskusteilbereich 72, 74 sind im Wesentlichen quaderförmig ausgebildet und über ein stegförmiges Verbindungselement 76 starr miteinander verbunden. Die mediale Gelenkfläche 26 bildet eine Oberseite des medialen Meniskusteilbereichs 72, die laterale Gelenkfläche 28 einer Oberseite des lateralen Meniskusteilbereichs 74. Das in Form eines Stegs 78 ausgebildete Verbindungselement 76 weist eine ebene, an der Tibiafläche 54 anliegende Verbindungselementfläche 80 auf.

Das Meniskusteil 16 umfasst eine Unterseite 82, welche zwei voneinander getrennte, ebene Flächenbereiche 84 beziehungsweise 86 aufweist. Die Flächenbereiche 84 und 86 bilden jeweils Unterseiten der Meniskusteilbereiche 72 und 74. Die Flächenbereiche 84 und 86 sind durch die Verbindungselementfläche 80 voneinander getrennt, bilden mit dieser zusammen jedoch die ebene, durchgehende Unterseite 82 des Meniskusteils 16.

Das Meniskusteil 16 ist medialseitig relativ zum Tibiateil 14 um eine Rotationsachse 88 drehbar gelagert. Die Rotationsachse 88 verläuft senkrecht zur Tibiaebene 56. Sie wird durch ein zwischen dem Tibiateil 14 und dem Meniskusteil 16 ausgebildetes Drehlager 90 definiert. Das Drehlager 90 umfasst erste und zweite, zusammenwirkende Lagerelemente 92, 94, wobei das eine am Tibiateil 14 und das andere am Meniskusteil 16 angeordnet oder ausgebildet ist. Eines der Lagerelemente 92, 94 ist in Form eines Vorsprungs 96 ausgebildet, das andere Lagerelement in Form einer korrespondierenden Ausnehmung 98. Bei dem in den Figuren dargestellten Ausführungsbeispiel der Kniegelenkendoprothese 10 ist der Vorsprung 96 vom Flächenbereich 84 in Form eines flachen Zylinders abstehend ausgebildet, welcher im Wesentlichen formschlüssig in die flache, hohlzylindrische Ausnehmung 98 eingreift. Die Lagerelemente 92, 94 sind somit rotationssymmetrisch zur Rotationsachse 88 ausgebildet. Der Vorsprung 96 und die Ausnehmung 98 weisen rotationssymmetrisch bezogen auf die Rotationsachse 88 ausgebildete erste und zweite Führungsflächen 100, 102 auf, die zur Führung einer Relativbewegung zwischen Tibiateil 14 und Meniskusteil 16 zusammenwirken. Die erste Führungsfläche 100 wird definiert durch eine in sich geschlossene ringförmige Wandfläche der Ausnehmung 98, die zweite Führungsfläche 102 durch eine ringförmige, in sich geschlossene Außenfläche des zylindrischen Vorsprungs 96. Die Führungsflächen 100 und 102 sind somit auch koaxial zur Rotationsachse ausgebildet. Durch die beschriebene Ausgestaltung des Vorsprungs und der Ausnehmung wird verhindert, dass das Meniskusteil 16 und das Tibiateil 14 relativ zueinander parallel zur Tibiaebene verschoben werden können. Bei dem in den Figuren dargestellten Ausführungsbeispiel ist lediglich eine Drehung um die Rotationsachse 88 des Meniskusteils 16 und des Tibiateils 14 relativ zueinander möglich.

Alternativ kann das Drehlager 90 auch in Form eines in den Figuren nicht dargestellten Kugelgelenklagers ausgebildet sein, welches erste und zweite Kugelgelenkflächen umfasst, wobei eine der Kugelgelenkflächen hohlkugelig und die andere Kugelgelenkfläche kugelig geformt ist. Vorzugsweise umfasst eines der Lagerelemente 92, 94 dann die hohlkugelige Kugelgelenkfläche und das andere die kugelige Kugelgelenkfläche. Beispielsweise könnte die hohlkugelige Kugelgelenkfläche am Tibiateil 14 ausgebildet sein, denkbare wäre es auch, die hohlkugelige Kugelgelenkfläche am Meniskusteil 16 auszubilden. Um eine optimale Führung der Lagerelemente 92 und 94 aneinander zu erhalten, sind bei einem Kugelgelenklager die ersten und zweiten Kugelgelenkflächen derart ausgebildet, dass sie identische oder im Wesentlichen identische Krümmungsradien aufweisen.

Die Kniegelenkendoprothese 10 umfasst ferner eine Rotationsführungseinrichtung 104 zum Erzwingen einer Rotationsbewegung des Meniskusteils 16 relativ zum Tibiateil 14 um die Rotationsachse 88 infolge einer Schwenkbewegung des Femurteils 12 und des Tibiateils 14 relativ zueinander um eine quer zur Rotationsachse 88 verlaufende Schwenkachse 106. Die Rotationsführungseinrichtung 104 umfasst zusammenwirkende erste und zweite Führungselemente 108, 110, die einerseits am Tibiateil 14 und andererseits am Femurteil 12 angeordnet oder ausgebildet sind. Das erste und das zweite Führungselement 108, 110 umfassen erste und zweite Führungselementflächen 112, 114, die mindestens ab einem bestimmten Beugungswinkel zwischen Femur 50 und Tibia 66 mindestens teilweise aneinander anliegen.

Das zweite Führungselement 110 ist im posterioren Bereich des Femurteils 12 ausgebildet. Es wird im Wesentlichen gebildet durch das Verbindungselement 44. Die zweite Führungselementfläche 114 weist im Wesentlichen in einer Richtung in den Zwischenraum 42 hinein. Es ist sonst am Femurteil 12 im Bereich zwischen den Kondylen 18, 20 ausgebildet. Die zweite Führungselementfläche weist mindestens einen in Richtung auf das Tibiateil 14 weisenden, konvexen Flächenbereich 116 auf.

Das erste Führungselement 108 ist im anterioren Bereich des Tibiateils 14 ausgebildet. Es ist in Form eines von der Tibiafläche 54 abstehenden Vorsprungs 118 ausgebildet, welcher auf der Tibiafläche 54 einen rechteckigen Flächenbereich 120 definiert. Die erste Führungselementfläche 112 ist von der Tibiafläche 54 beabstandet und konkav gekrümmt. Im unteren Bereich, insbesondere im Bereich des Flächenbereichs 120, ist das erste Führungselement 108 in sich spiegelsymmetrisch bezüglich einer vorzugsweise eine Sagittalebene definierenden Symmetrieebene 122 ausgebildet, die gleichzeitig eine Symmetrieebene der Platte 52 definiert. Die Symmetrieebene 122 ist senkrecht zur Tibiaebene 56 orientiert. Im posterioren Bereich, also im Bereich der ersten Führungselementfläche 112, ist das erste Führungselement 108 jedoch unsymmetrisch bezogen auf die Symmetrieebene 122 ausgebildet. Ein mediales Ende 124 des Vorsprungs 118 steht weiter in posteriorer Richtung vor als ein laterales Ende 126. Damit ist die erste Führungselementfläche ebenfalls unsymmetrisch bezüglich der Symmetrieebene 122 ausgebildet.

Die ersten und zweiten Führungselementflächen 112 und 114 sind in Form von Gleitflächen ausgebildet. Die erste Führungselementfläche 112 weist zudem einen in Richtung auf das Femurteil 12 weisenden konkaven Flächenbereich 128 auf, welcher an der zweiten Führungselementfläche 114 mindestens dann teilweise anliegt, wenn die ersten und zweiten Führungselemente 108 und 110 in Kontakt stehen und zusammenwirken.

Durch die besondere Ausgestaltung der ersten und zweiten Führungselemente 108 und 110 ist die Rotationsführungseinrichtung 104 insgesamt ausgebildet zum Erzwingen einer Rotation des Femurteils 12 relativ zum Tibiateil 14 um die Rotationsachse 88, wenn das Femurteil 12 um die Schwenkachse 106 relativ zum Meniskusteil 16 verschwenkt wird, also wenn Femur 50 und Tibia 66 aus einer Streck- oder Extensionsstellung, in welcher deren Längsachsen im Wesentlichen parallel zueinander ausgerichtet sind, in eine Beugestellung gebracht werden. Man spricht hier auch von einer Flexionsbewegung. Die Rotationsführungseinrichtung 104 kann insbesondere ausgebildet sein zum Erzwingen einer Abrollbewegung des Femurteils 12 und des Meniskusteils 16 aneinander. Dies kann insbesondere dadurch erreicht werden, dass eine Krümmung der medialen und/oder lateralen Gelenkflächen 26, 28 größer ist als eine Krümmung der medialen und lateralen Kondylenflächen 22, 24. Werden die Krümmungsradien dieser Flächen aneinander angepasst, so kann die Rotationsführungseinrichtung 104 auch derart ausgebildet werden, dass sie eine Gleitbewegung, oder sogar ausschließlich eine solche, des Femurteils und des Meniskusteils 16 relativ zueinander ermöglicht. Ferner kann die Rotationsführungseinrichtung 104 auch derart ausgebildet sein, dass sie eine überlagerte Gleit-/ Abrollbewegung des Femurteils 12 und des Meniskusteils 16 relativ zueinander ermöglicht. Dies kann beispielsweise erreicht werden durch entsprechend vorgesehene, nicht identische Krümmungsradien der Kondylenflächen 22, 24 und der Gelenkflächen 26, 28.

Die ersten und zweiten Führungselemente 108, 110 sind aufgrund ihrer besonders geformten ersten und zweiten Führungselementflächen 112, 114 ausgebildet zum Definieren eines Rotationswinkels einer Rotations- oder Drehbewegung des Meniskusteils 16 und des Tibiateils 14 relativ zueinander um die Rotationsachse 88 in Abhängigkeit eines Flexionswinkels zwischen Femurteil 12 und Tibiateil 14. Mit anderen Worten bedeutet dies, dass das Meniskusteil 16 mit seinem lateralen Meniskusteilbereich 74 um so weiter in posteriorer Richtung verdreht wird, je größer ein Beuge- oder Flexionswinkel zwischen Femurteil 12 und Tibiateil 14 ist, wobei der Beugewinkel beispielsweise ausgehend von einem gestreckten Kniegelenk gemessen werden kann. Aufgrund des Abstands zwischen den ersten und zweiten Führungselementflächen 112 und 114 bei dem in den Figuren dargestellten Ausführungsbeispiel in der Streckoder Extensionsstellung, setzt eine Zwangsrotation um die Rotationsachse 88 erst ab einem bestimmten oder minimalen Beugewinkel ein. Der minimale oder erforderliche Beugewinkel liegt vorzugsweise in einem Bereich zwischen 30° und 60°.

Zum Sichern des Meniskusteils 16 am Tibiateil 14 in einer Verbindungsstellung, in welcher das Meniskusteil 16 und das Tibiateil 14 um die Rotationsachse 88 rotierbar gelagert sind und nicht voneinander getrennt werden können ist eine insgesamt mit dem Bezugszeichen 130 versehene Sicherungseinrichtung vorgesehen. Sie umfasst erste und zweite, zusammenwirkende Sicherungselemente 132 und 134, welche einerseits am Meniskusteil 16 und andererseits am Tibiateil 14 ausgebildet sind. Die ersten und zweiten Sicherungselemente 132 und 134 weisen quer zur Rotationsachse 88 verlaufende Anschlagflächen 136 und 138 auf zum Verhindern einer Bewegung des Meniskusteils 16 und des Tibiateils 14 in der Verbindungsstellung voneinander weg. Das erste Sicherungselement 132 umfasst einen am Tibiateil 14 angeordneten oder ausgebildeten ersten Rückhaltevorsprung 140. Das zweite Sicherungselement 134 umfasst einen am Meniskusteil 16 angeordneten zweiten Rückhaltevorsprung 142. Der erste Rückhaltevorsprung 140 ist an eine Ausnehmung 144 des ersten Führungselements 108 angrenzend ausgebildet. Insgesamt umfasst das Führungselement 108 den ersten Rückhaltevorsprung 140. Dieser umfasst die erste Anschlagfläche 136, welche von der Tibiafläche 154 beziehungsweise der Tibiaebene 56 beabstandet ist.

Das Verbindungselement 76 bildet den zweiten Rückhaltevorsprung 142. Eine Oberseite des Verbindungselements 76 bildet die zweite Anschlagfläche 138 der Sicherungseinrichtung 130.

Die Ausnehmung 144 am ersten Führungselement 108 ist derart dimensioniert, dass das Verbindungselement 76 in die Ausnehmung 144 eingreifen kann. Eine in posteriorer Richtung weisende Seitenfläche 146 des ersten Führungselements 108, welche die Ausnehmung 144 begrenzt, bildet einen Rotationsbegrenzungsanschlag 148 zum Begrenzen einer Bewegung des lateralen Meniskusteilbereichs 74 in anteriorer Richtung. In der Extensionsstellung verläuft dann das Verbindungselement 76 vorzugsweise senkrecht zur Symmetrieebene 122 und ist vorzugsweise in dieser Stellung zur Symmetrieebene 122 spiegelsymmetrisch ausgebildet und ausgerichtet. Wenn das Verbindungselement 76 mindestens teilweise in die Ausnehmung 144 eintaucht oder eingreift, nehmen das Meniskusteil 16 und das Tibiateil 14 die bereits beschriebene Verbindungsstellung ein. Sie können dann relativ zueinander nicht in einer Richtung parallel zur Rotationsachse 88 bewegt werden. Das Meniskusteil 16 und das Tibiateil 14 sind jedoch von der Verbindungsstellung in eine Montagestellung bringbar, in welcher die ersten und zweiten Anschlagflächen 136 und 138 außer Eingriff stehen. Dies wird erreicht durch Rotieren des Meniskusteils 16 relativ zum Tibiateil 14 um die Rotationsachse 88 um einen Lösewinkel. In der Verbindungsstellung überlappen senkrechte Projektionen der Anschlagflächen 136, 138 auf die Tibiafläche 54 einander und definieren so einen projizierten Flächenabschnitt auf der Tibiafläche 54. So lange der Flächeninhalt dieses Flächenabschnitts größer als Null ist, nehmen das Meniskusteil 16 und das Tibiateil 14 die Verbindungsstellung ein. In der Verbindungsstellung sind sie relativ zueinander jedoch frei beweglich um die Rotationsachse 88.

Des Weiteren sei angemerkt, dass die erste Führungselementfläche 112 bezogen auf die erste Anschlagfläche 136 in posteriorer Richtung versetzt ausgebildet ist. Bei dem in den Figuren dargestellten ersten Führungselement 108 bilden die erste Führungselementfläche 112 und die erste Anschlagfläche 136 eine gemeinsame Kante 150 aus.

Sowohl das Femurteil 12 als auch das Tibiateil 14 sind vorzugsweise einstückig ausgebildet. Auch das Meniskusteil 16 ist bei dem beschriebenen Ausführungsbeispiel einstückig ausgebildet. Insbesondere das Femurteil 12 und das Tibiateil 14 können optional auch in Form modularer Prothesenteile ausgebildet sein. Wie bereits weiter oben beschrieben, können sowohl das Femurteil 12 als auch das Meniskusteil 16 bei alternativen Ausführungsformen mit modularen Schäften ausgestattet sein, die in Länge und Durchmesser an die jeweilige Physiologie des Patienten angepasst werden können.

Das Femurteil 12 und das Tibiateil 14 sind vorzugsweise aus einem Instrumentenstahl hergestellt, das Meniskusteil 16 aus einem hochabriebfesten Kunststoff.

24 des Meniskusteils 16 sind vom Femurteil 12 weg weisend im Wesentlichen konvex gekrümmt und zum Abgleiten beziehungsweise Abrollen an medialen und lateralen Gelenkflächen 26, 28 des Meniskusteils 16 ausgebildet, an welchen sie mindestens teilweise anliegen.

Für eine verbesserte Führung einer Relativbewegung des Femurteils 12 und des Meniskusteils 16 aneinander weisen die mediale und die laterale Kondylenfläche 22, 24 vorzugsweise jeweils einen konkav gekrümmten Kondylenflächenbereich 30, 32 auf, welche im Wesentlichen korrespondierend zum Zusammenwirken mit konvex gekrümmten medialen und lateralen Kondylenflächenbereichen 34, 36 der ansonsten im Wesentlichen vom Meniskusteil 16 weg weisend konkav gekrümmten medialen und lateralen Gelenkflächen 26, 28 ausgebildet sind. Krümmungsradien der medialen und/oder lateralen Gelenkflächen 26, 28 sind vorzugsweise größer als Krümmungsradien der medialen und/oder lateralen Kondylenflächen 22, 24 ausgebildet, so dass nicht nur eine Gleitbewegung zwischen dem Femurteil 12 und dem Meniskusteil 16 realisierbar ist, sondern gleichzeitig auch eine Abrollbewegung, die der Gleitbewegung insbesondere überlagert sein kann.

Die Kondylen 18 und 20 sind an ihrem vorderen oder anterioren Ende 38 miteinander verbunden. Zwischen ihrem anterioren Ende 38 und einem posterioren Ende 40 ist ein Spalt oder Zwischenraum 42 zwischen den beiden voneinander beabstandeten Kondylen 18 und 20 ausgebildet. Im Bereich ihrer posterioren Enden 40 sind die Kondylen 18 und 20 über ein Verbindungselement 44 miteinander fest verbunden.

Die Kondylenflächen 22 und 24 bilden im Wesentlichen eine Vorder- oder Außenseite des Femurteils 12. In entgegengesetzter Richtung weisend, das heißt auf einer Rückseite oder Innenseite der Kondylen 18 und 20 sind jeweils Anlageflächen 46 ausgebildet, welche insbesondere mehrere relativ zueinander geneigte, ebene Anlagenflächenbereiche 48 aufweisen können. Zum Fixieren des Femurteils 12 an einem Femur 50 eines Patienten wird der Femur 50 entsprechend der Rückseite des Femurteils 12 präpariert, das heißt es werden in den Figuren nicht näher dargestellte Anlageflächen am Femur 50 präpariert, die zu den Anlagenflächen 46 korrespondieren, um das Femurteil 12 beispielsweise mit Knochenzement am Femur 50 festzulegen. Optional können auch nicht dargestellte Knochenschrauben zum Einsatz kommen, um das Femurteil 12 alternativ oder zusätzlich am Femur 50 zu sichern.

In den Figuren ebenfalls nicht dargestellt sind alternative Ausführungsformen von Femurteilen 12, die zusätzlich einen oder mehrere vom Femurteil 12 abstehende Schaftabschnitte aufweisen, die in entsprechende Ausnehmungen, die zuvor am Femur 50 präpariert wurden, eingesetzt und mittels Knochenzement und/oder Befestigungselementen, wie beispielsweise Knochenschrauben, festgelegt werden können. Derartige Schäfte oder Schaftabschnitte können insbesondere modular ausgebildet sein, um ihre Länge entsprechend optimiert an eine Physiologie des Patienten anpassen zu können.

Das Tibiateil 14 umfasst eine Platte 52, welche eine in Richtung auf das Meniskusteil 16 weisende Tibiafläche 54 aufweist, die eine Tibiaebene 56 definiert. Somit ist die Tibiafläche 54 eben. Die Tibiafläche 54 bildet eine Oberseite 58 der Platte 52, die in einer Draufsicht im Wesentlichen nierenförmig geformt ist. Von einer Unterseite 60 der Platte 52 ist ein Schaft oder Schaftabschnitt 62 abstehend angeordnet oder ausgebildet. Ein distales Ende 64 desselben kann optional mit Verlängerungselementen verbunden werden, die in eine entsprechend vorbereitete Kavität 68 einer Tibia 66 eingesetzt werden können. Zum Anlegen der Platte 52 wird die Tibia 66 teilreseziert und eine ebene Anlagefläche 70 präpariert, an welcher die Unterseite 58 im Wesentlichen großflächig anliegt.

Das Meniskusteil 16 umfasst einen medialen Meniskusteilbereich 72 und einen lateralen Meniskusteilbereich 74. Der laterale und der mediale Meniskusteilbereich 72, 74 sind im Wesentlichen quaderförmig ausgebildet und über ein stegförmiges Verbindungselement 76 starr miteinander verbunden. Die mediale Gelenkfläche 26 bildet eine Oberseite des medialen Meniskusteilbereichs 72, die laterale Gelenkfläche 28 einer Oberseite des lateralen Meniskusteilbereichs 74. Das in Form eines Stegs 78 ausgebildete Verbindungselement 76 weist eine ebene, an der Tibiafläche 54 anliegende Verbindungselementfläche 80 auf.

Das Meniskusteil 16 umfasst eine Unterseite 82, welche zwei voneinander getrennte, ebene Flächenbereiche 84 beziehungsweise 86 aufweist. Die Flächenbereiche 84 und 86 bilden jeweils Unterseiten der Meniskusteilbereiche 72 und 74. Die Flächenbereiche 84 und 86 sind durch die Verbindungselementfläche 80 voneinander getrennt, bilden mit dieser zusammen jedoch die ebene, durchgehende Unterseite 82 des Meniskusteils 16.

Das Meniskusteil 16 ist medialseitig relativ zum Tibiateil 14 um eine Rotationsachse 88 drehbar gelagert. Die Rotationsachse 88 verläuft senkrecht zur Tibiaebene 56. Sie wird durch ein zwischen dem Tibiateil 14 und dem Meniskusteil 16 ausgebildetes Drehlager 90 definiert. Das Drehlager 90 umfasst erste und zweite, zusammenwirkende Lagerelemente 92, 94, wobei das eine am Tibiateil 14 und das andere am Meniskusteil 16 angeordnet oder ausgebildet ist. Eines der Lagerelemente 92, 94 ist in Form eines Vorsprungs 96 ausgebildet, das andere Lagerelement in Form einer korrespondierenden Ausnehmung 98. Bei dem in den Figuren dargestellten Ausführungsbeispiel der Kniegelenkendoprothese 10 ist der Vorsprung 96 vom Flächenbereich 84 in Form eines flachen Zylinders abstehend ausgebildet, welcher im Wesentlichen formschlüssig in die flache, hohlzylindrische Ausnehmung 98 eingreift. Die Lagerelemente 92, 94 sind somit rotationssymmetrisch zur Rotationsachse 88 ausgebildet. Der Vorsprung 96 und die Ausnehmung 98 weisen rotationssymmetrisch bezogen auf die Rotationsachse 88 ausgebildete erste und zweite Führungsflächen 100, 102 auf, die zur Führung einer Relativbewegung zwischen Tibiateil 14 und Meniskusteil 16 zusammenwirken. Die erste Führungsfläche 100 wird definiert durch eine in sich geschlossene ringförmige Wandfläche der Ausnehmung 98, die zweite Führungsfläche 102 durch eine ringförmige, in sich geschlossene Außenfläche des zylindrischen Vorsprungs 96. Die Führungsflächen 100 und 102 sind somit auch koaxial zur Rotationsachse ausgebildet. Durch die beschriebene Ausgestaltung des Vorsprungs und der Ausnehmung wird verhindert, dass das Meniskusteil 16 und das Tibiateil 14 relativ zueinander parallel zur Tibiaebene verschoben werden können. Bei dem in den Figuren dargestellten Ausführungsbeispiel ist lediglich eine Drehung um die Rotationsachse 88 des Meniskusteils 16 und des Tibiateils 14 relativ zueinander möglich.

Alternativ kann das Drehlager 90 auch in Form eines in den Figuren nicht dargestellten Kugelgelenklagers ausgebildet sein, welches erste und zweite Kugelgelenkflächen umfasst, wobei eine der Kugelgelenkflächen hohlkugelig und die andere Kugelgelenkfläche kugelig geformt ist. Vorzugsweise umfasst eines der Lagerelemente 92, 94 dann die hohlkugelige Kugelgelenkfläche und das andere die kugelige Kugelgelenkfläche. Beispielsweise könnte die hohlkugelige Gelenkfläche am Tibiateil 14 ausgebildet sein, denkbare wäre es auch, die hohlkugelige Kugelgelenkfläche am Meniskusteil 16 auszubilden. Um eine optimale Führung der Lagerelemente 92 und 94 aneinander zu erhalten, sind bei einem Kugelgelenklager die ersten und zweiten Kugelgelenkflächen derart ausgebildet, dass sie identische oder im Wesentlichen identische Krümmungsradien aufweisen.

Die Kniegelenkendoprothese 10 umfasst ferner eine Rotationsführungseinrichtung 104 zum Erzwingen einer Rotationsbewegung des Meniskusteils 16 relativ zum Tibiateil 14 um die Rotationsachse 88 infolge einer Schwenkbewegung des Femurteils 12 und des Tibiateils 14 relativ zueinander um eine quer zur Rotationsachse 88 verlaufende Schwenkachse 106. Die Rotationsführungseinrichtung 104 umfasst zusammenwirkende erste und zweite Führungselemente 108, 110, die einerseits am Tibiateil 14 und andererseits am Femurteil 12 angeordnet oder ausgebildet sind. Das erste und das zweite Führungselement 108, 110 umfassen erste und zweite Führungselementflächen 112, 114, die mindestens ab einem bestimmten Beugungswinkel zwischen Femur 50 und Tibia 66 mindestens teilweise aneinander anliegen.

Das zweite Führungselement 110 ist im posterioren Bereich des Femurteils 12 ausgebildet. Es wird im Wesentlichen gebildet durch das Verbindungselement 44. Die zweite Führungselementfläche 114 weist im Wesentlichen in einer Richtung in den Zwischenraum 42 hinein. Es ist sonst am Femurteil 12 im Bereich zwischen den Kondylen 18, 20 ausgebildet. Die zweite Führungselementfläche weist mindestens einen in Richtung auf das Tibiateil 14 weisenden, konvexen Flächenbereich 116 auf.

Das erste Führungselement 108 ist im anterioren Bereich des Tibiateils 14 ausgebildet. Es ist in Form eines von der Tibiafläche 54 abstehenden Vorsprungs 118 ausgebildet, welcher auf der Tibiafläche 54 einen rechteckigen Flächenbereich 120 definiert. Die erste Führungselementfläche 112 ist von der Tibiafläche 54 beabstandet und konkav gekrümmt. Im unteren Bereich, insbesondere im Bereich des Flächenbereichs 120, ist das erste Führungselement 108 in sich spiegelsymmetrisch bezüglich einer vorzugsweise eine Sagittalebene definierenden Symmetrieebene 122 ausgebildet, die gleichzeitig eine Symmetrieebene der Platte 52 definiert. Die Symmetrieebene 122 ist senkrecht zur Tibiaebene 56 orientiert. Im posterioren Bereich, also im Bereich der ersten Führungselementfläche 112, ist das erste Führungselement 108 jedoch unsymmetrisch bezogen auf die Symmetrieebene 122 ausgebildet. Ein mediales Ende 124 des Vorsprungs 118 steht weiter in posteriorer Richtung vor als ein laterales Ende 126. Damit ist die erste Führungselementfläche ebenfalls unsymmetrisch bezüglich der Symmetrieebene 122 ausgebildet.

Die ersten und zweiten Führungselementflächen 112 und 114 sind in Form von Gleitflächen ausgebildet. Die erste Führungselementfläche 112 weist zudem einen in Richtung auf das Femurteil 12 weisenden konkaven Flächenbereich 128 auf, welcher an der zweiten Führungselementfläche 114 mindestens dann teilweise anliegt, wenn die ersten und zweiten Führungselemente 108 und 110 in Kontakt stehen und zusammenwirken.

Durch die besondere Ausgestaltung der ersten und zweiten Führungselemente 108 und 110 ist die Rotationsführungseinrichtung 104 insgesamt ausgebildet zum Erzwingen einer Rotation des Femurteils 12 relativ zum Tibiateil 14 um die Rotationsachse 88, wenn das Femurteil 12 um die Schwenkachse 106 relativ zum Meniskusteil 116 verschwenkt wird, also wenn Femur 50 und Tibia 66 aus einer Streck- oder Extensionsstellung, in welcher deren Längsachsen im Wesentlichen parallel zueinander ausgerichtet sind, in eine Beugestellung gebracht werden. Man spricht hier auch von einer Flexionsbewegung. Die Rotationsführungseinrichtung 104 kann insbesondere ausgebildet sein zum Erzwingen einer Abrollbewegung des Femurteils 12 und des Meniskusteils 16 aneinander. Dies kann insbesondere dadurch erreicht werden, dass eine Krümmung der medialen und/oder lateralen Gelenkflächen 26, 28 größer ist als eine Krümmung der medialen und lateralen Kondylenflächen 22, 24. Werden die Krümmungsradien dieser Flächen aneinander angepasst, so kann die Rotationsführungseinrichtung 104 auch derart ausgebildet werden, dass sie eine Gleitbewegung, oder sogar ausschließlich eine solche, des Femurteils und des Meniskusteils 16 relativ zueinander ermöglicht. Ferner kann die Rotationsführungseinrichtung 104 auch derart ausgebildet sein, dass sie eine überlagerte Gleit-/ Abrollbewegung des Femurteils 12 und des Meniskusteils 16 relativ zueinander ermöglicht. Dies kann beispielsweise erreicht werden durch entsprechend vorgesehene, nicht identische Krümmungsradien der Kondylenflächen 42, 24 und der Gelenkflächen 26, 28.

Die ersten und zweiten Führungselemente 108, 110 sind aufgrund ihrer besonders geformten ersten und zweiten Führungselementflächen 112, 114 ausgebildet zum Definieren eines Rotationswinkels einer Rotations- oder Drehbewegung des Meniskusteils 16 und des Tibiateils 14 relativ zueinander um die Rotationsachse 88 in Abhängigkeit eines Flexionswinkels zwischen Femurteil 12 und Tibiateil 14. Mit anderen Worten bedeutet dies, dass das Meniskusteil 16 mit seinem lateralen Meniskusteilbereich 74 um so weiter in posteriorer Richtung verdreht wird, je größer ein Beuge- oder Flexionswinkel zwischen Femurteil 12 und Tibiateil 14 ist, wobei der Beugewinkel beispielsweise ausgehend von einem gestreckten Kniegelenk gemessen werden kann. Aufgrund des Abstands zwischen den ersten und zweiten Führungselementflächen 112 und 114 bei dem in den Figuren dargestellten Ausführungsbeispiel in der Streckoder Extensionsstellung, setzt eine Zwangsrotation um die Rotationsachse 88 erst ab einem bestimmten oder minimalen Beugewinkel ein. Der minimale oder erforderliche Beugewinkel liegt vorzugsweise in einem Bereich zwischen 30° und 60°.

Zum Sichern des Meniskusteils 16 am Tibiateil 14 in einer Verbindungsstellung, in welcher das Meniskusteil 16 und das Tibiateil 14 um die Rotationsachse 88 rotierbar gelagert sind und nicht voneinander getrennt werden können ist eine insgesamt mit dem Bezugszeichen 130 versehene Sicherungseinrichtung vorgesehen. Sie umfasst erste und zweite, zusammenwirkende Sicherungselemente 132 und 134, welche einerseits am Meniskusteil 16 und andererseits am Tibiateil 14 ausgebildet sind. Die ersten und zweiten Sicherungselemente 132 und 134 weisen quer zur Rotationsachse 88 verlaufende Anschlagflächen 136 und 138 auf zum Verhindern einer Bewegung des Meniskusteils 16 und des Tibiateils 14 in der Verbindungsstellung voneinander weg. Das erste Sicherungselement 132 umfasst einen am Tibiateil 14 angeordneten oder ausgebildeten ersten Rückhaltevorsprung 140. Das zweite Sicherungselement 134 umfasst einen am Meniskusteil 16 angeordneten zweiten Rückhaltevorsprung 142. Der erste Rückhaltevorsprung 140 ist an eine Ausnehmung 144 des ersten Führungselements 108 angrenzend ausgebildet. Insgesamt umfasst das Führungselement 108 den ersten Rückhaltevorsprung 140. Dieser umfasst die erste Anschlagfläche 136, welche von der Tibiafläche 154 beziehungsweise der Tibiaebene 56 beabstandet ist.

Das Verbindungselement 76 bildet den zweiten Rückhaltevorsprung 142. Eine Oberseite des Verbindungselements 76 bildet die zweite Anschlagfläche 138 der Sicherungseinrichtung 130.

Die Ausnehmung 144 am ersten Führungselement 108 ist derart dimensioniert, dass das Verbindungselement 76 in die Ausnehmung 144 eingreifen kann. Eine in posteriorer Richtung weisende Seitenfläche 146 des ersten Führungselements 108, welche die Ausnehmung 144 begrenzt, bildet einen Rotationsbegrenzungsanschlag 148 zum Begrenzen einer Bewegung des lateralen Meniskusteilbereichs 74 in anteriorer Richtung. In der Extensionsstellung verläuft dann das Verbindungselement 76 vorzugsweise senkrecht zur Symmetrieebene 122 und ist vorzugsweise in dieser Stellung zur Symmetrieebene 122 spiegelsymmetrisch ausgebildet und ausgerichtet. Wenn das Verbindungselement 76 mindestens teilweise in die Ausnehmung 144 eintaucht oder eingreift, nehmen das Meniskusteil 16 und das Tibiateil 14 die bereits beschriebene Verbindungsstellung ein. Sie können dann relativ zueinander nicht in einer Richtung parallel zur Rotationsachse 88 bewegt werden. Das Meniskusteil 16 und das Tibiateil 14 sind jedoch von der Verbindungsstellung in eine Montagestellung bringbar, in welcher die ersten und zweiten Anschlagflächen 136 und 138 außer Eingriff stehen. Dies wird erreicht durch Rotieren des Meniskusteils 16 relativ zum Tibiateil 14 um die Rotationsachse 88 um einen Lösewinkel. In der Verbindungsstellung überlappen senkrechte Protektionen der Anschlagflächen 136, 138 auf die Tibiafläche 54 einander und definieren so einen projizierten Flächenabschnitt auf der Tibiafläche 54. So lange der Flächeninhalt dieses Flächenabschnitts größer als Null ist, nehmen das Meniskusteil 16 und das Tibiateil 14 die Verbindungsstellung ein. In der Verbindungsstellung sind sie relativ zueinander jedoch frei beweglich um die Rotationsachse 88.

Des Weiteren sei angemerkt, dass die erste Führungselementfläche 112 bezogen auf die erste Anschlagfläche 136 in posteriorer Richtung versetzt ausgebildet ist. Bei dem in den Figuren dargestellten ersten Führungselement 108 bilden die erste Führungselementfläche 112 und die erste Anschlagfläche 136 eine gemeinsame Kante 150 aus.

Sowohl das Femurteil 12 als auch das Tibiateil 14 sind vorzugsweise einstückig ausgebildet. Auch das Meniskusteil 16 ist bei dem beschriebenen vorzugsweise Ausführungsteil einstückig ausgebildet. Insbesondere das Femurteil 12 und das Tibiateil 14 können optional auch in Form modularer Prothesenteile ausgebildet sein. Wie bereits weiter oben beschrieben, können sowohl das Femurteil 12 als auch das Meniskusteil 16 bei alternativen Ausführungsformen mit modularen Schäften ausgestattet sein, die in Länge und Durchmesser an die jeweilige Physiologie des Patienten angepasst werden können.

Das Femurteil 12 und das Tibiateil 14 sind vorzugsweise aus einem Instrumentenstahl hergestellt, das Meniskusteil 16 aus einem hochabriebfesten Kunststoff.

## Patentansprüche

1. Kniegelenkendoprothese (10) mit einem Femurteil (12), einem Tibiateil (14) und einem zwischen dem Femurteil (12) und dem Tibiateil (14) gelagerten Meniskusteil (16), wobei das Meniskusteil (16) am Tibiateil (14) um eine medialseitig verlaufende Rotationsachse (88) rotierbar gelagert ist, **dadurch gekennzeichnet, dass** eine Rotationsführungseinrichtung (104) vorgesehen ist zum Erzwingen einer Rotationsbewegung des Meniskusteils (16) relativ zum Tibiateil (14) um die Rotationsachse (88) infolge einer Schwenkbewegung des Femurteils (12) und des Tibiateils (14) relativ zueinander um eine quer zur Rotationsachse (88) verlaufende Schwenkachse (106) und dass die Rotationsführungseinrichtung (104) zusammenwirkende erste und zweite Führungselemente (108, 110) umfasst, die einerseits am Femurteil (12) und andererseits am Tibiateil (14) angeordnet oder ausgebildet sind.

2. Kniegelenkendoprothese nach Anspruch 1, **dadurch gekennzeichnet, dass** das Femurteil (12) eine mediale und eine laterale Kondyle (18, 20) umfasst, welche eine mediale und eine laterale Kondylenfläche (22, 24) aufweisen, welches Meniskusteil (16) eine mediale und eine laterale Gelenkfläche (26, 28) aufweist, an welchen die medialen und lateralen Kondylenflächen (22, 24) mindestens teilweise anliegen.

3. Kniegelenkendoprothese nach Anspruch 2, **dadurch gekennzeichnet, dass** die mediale und/oder die laterale Kondylenfläche (22, 24) einen konkav gekrümmten Kondylenflächenbereich (30, 32) und dass die mediale und/oder die laterale Gelenkfläche (26, 28) einen zur medialen und/oder lateralen Kondylenfläche (22, 24) korrespondierenden konvex gekrümmten Gelenkflächenbereich (34, 36) umfassen.

4. Kniegelenkendoprothese nach Anspruch 2 oder 3, **dadurch gekennzeichnet, dass** Krümmungsradien der medialen und/oder lateralen Gelenkflächen (26, 28) größer sind als Krümmungsradien der medialen und/oder lateralen Kondylenflächen (22, 24).

5. Kniegelenkendoprothese nach einem der voranstehenden Ansprüche, **dadurch gekennzeichnet, dass** die Rotationsachse (88) durch ein zwischen dem Tibiateil (14) und dem Meniskusteil (16) ausgebildetes Drehlager (90) definiert wird.

6. Kniegelenkendoprothese nach Anspruch 5, **dadurch gekennzeichnet, dass** das Drehlager (90) erste und zweite, zusammenwirkende Lagerelemente (92, 94) umfasst, welche einerseits am Tibiateil (14) und andererseits am Meniskusteil (16) angeordnet oder ausgebildet sind.

7. Kniegelenkendoprothese nach Anspruch 6, **dadurch gekennzeichnet, dass** eines der Lagerelemente (94) zylindrisch und das andere Lagerelement (92) hohlzylindrisch geformt ist.

8. Kniegelenkendoprothese nach einem der voranstehenden Ansprüche, **dadurch gekennzeichnet, dass** die Rotationsführungseinrichtung (104) ausgebildet ist zum Erzwingen einer Abrollbewegung des Femurteils (12) und des Meniskusteils (16) aneinander.

9. Kniegelenkendoprothese nach einem der voranstehenden Ansprüche, **dadurch gekennzeichnet, dass** die ersten und zweiten Führungselemente (108, 110) ausgebildet sind zum Definieren eines Rotationswinkels einer Rotationsbewegung des Meniskusteils (16) und des Tibiateils (14) um die Rotationsachse (88) in Abhängigkeit eines Flexionswinkels zwischen Femurteil (12) und Tibiateil (14).

10. Kniegelenkendoprothese nach einem der voranstehenden Ansprüche, **dadurch gekennzeichnet, dass** das erste Führungselement (108) im anterioren Bereich des Tibiateils (14) ausgebildet ist.

11. Kniegelenkendoprothese nach einem der voranstehenden Ansprüche, **dadurch gekennzeichnet, dass** das Meniskusteil (16) einen medialen Meniskusteilbereich (72) und einen lateralen Meniskusteilbereich (74) umfasst und dass der laterale und der mediale Meniskusteilbereich (72, 74) durch ein Verbindungselement (76) miteinander verbunden sind.

12. Kniegelenkendoprothese nach Anspruch 11, **dadurch gekennzeichnet, dass** das Verbindungselement (76) in Form eines Stegs (78) ausgebildet ist.

13. Kniegelenkendoprothese nach einem der voranstehenden Ansprüche, **dadurch gekennzeichnet, dass** das erste Führungselement (108) von einer in Richtung auf das Meniskusteil (16) weisenden Tibiafläche (56) abstehend ausgebildet ist.

14. Kniegelenkendoprothese nach einem der voranstehenden Ansprüche, **gekennzeichnet durch** eine Sicherungseinrichtung (130) zum Sichern des Meniskusteils (16) am Tibiateil (14) in einer Verbindungsstellung, in welcher das Meniskusteil (16) und das Tibiateil (14) um die Rotationsachse (88) rotierbar gelagert sind.

15. Kniegelenkendoprothese nach einem der voranstehenden Ansprüche, **gekennzeichnet durch** einen Rotationsbegrenzungsanschlag (148) zum Begrenzen einer Bewegung der lateralen Gelenkfläche (28) in anteriorer Richtung.

## Claims

1. Knee joint endoprosthesis (10) comprising a femoral component (12), a tibial component (14) and a meniscal component (16) mounted between the femoral component (12) and the tibial component (14), wherein the meniscal component (16) is mounted on the tibial component (14) for rotation about an axis of rotation (88) extending at the medial side, **characterized in that** a rotation guiding device (104) is provided to force rotational movement of the meniscal component (16) relative to the tibial component (14) about the axis of rotation (88) as a result of pivotal movement of the femoral component (12) and the tibial component (14) relative to each other about a pivot axis (106) extending transversely to the axis of rotation (88), and **in that** said rotation guiding device (104) has interacting first and second guiding elements (108, 110) arranged or formed, on the one hand, on the femoral component (12) and, on the other hand, on the tibial component (14).

2. Knee joint endoprosthesis in accordance with claim 1, **characterized in that** the femoral component (12) comprises a medial condyle and a lateral condyle (18, 20), which have a medial condylar surface and a lateral condylar surface (22, 24), which meniscal component (16) comprises a medial joint surface and a lateral joint surface (26, 28) on which the medial and lateral condylar surfaces (22, 24) bear at least partially.

3. Knee joint endoprosthesis in accordance with claim 2, **characterized in that** the medial and/or the lateral condylar surface (22, 24) comprises a concavely curved condylar surface region (30, 32), and **in that** the medial and/or the lateral joint surface (26, 28) comprises a convexly curved joint surface region (34, 36) corresponding to the medial and/or the lateral condylar surface (22, 24).

4. Knee joint endoprosthesis in accordance with claim 2 or 3, **characterized in that** radii of curvature of the medial and/or lateral joint surfaces (26, 28) are larger than radii of curvature of the medial and/or lateral condylar surfaces (22, 24).

5. Knee joint endoprosthesis in accordance with any one of the preceding claims, **characterized in that** the axis of rotation (88) is defined by a rotary bearing (90) formed between the tibial component (14) and the meniscal component (16).

6. Knee joint endoprosthesis in accordance with claim 5, **characterized in that** the rotary bearing (90) comprises interacting first and second bearing elements (92, 94) which are arranged or formed, on the one hand, on the tibial component (14) and, on the other hand, on the meniscal component (16).

7. Knee joint endoprosthesis in accordance with claim 6, **characterized in that** one of the bearing elements (94) is of cylindrical shape and the other bearing element (92) is of hollow-cylindrical shape.

8. Knee joint endoprosthesis in accordance with any one of the preceding claims, **characterized in that** the rotation guiding device (104) is configured to force rolling movement of the femoral component (12) and the meniscal component (16) on each other.

9. Knee joint endoprosthesis in accordance with any one of the preceding claims, **characterized in that** the first and second guiding elements (108, 110) are configured to define an angle of rotation of rotational movement of the meniscal component (16) and the tibial component (14) about the axis of rotation (88) in dependence upon a flexion angle between femoral component (12) and tibial component (14).

10. Knee joint endoprosthesis in accordance with any one of the preceding claims, **characterized in that** the first guiding element (108) is formed in the anterior region of the tibial component (14).

11. Knee joint endoprosthesis in accordance with any one of the preceding claims, **characterized in that** the meniscal component (16) comprises a medial meniscal component region (72) and a lateral meniscal component region (74), and **in that** the lateral meniscal component region and the medial meniscal component region (72, 74) are connected to each other by a connection element (76).

12. Knee joint endoprosthesis in accordance with claim 11, **characterized in that** the connection element (76) takes the form of a web (78).

13. Knee joint endoprosthesis in accordance with any one of the preceding claims, **characterized in that** the first guiding element (108) is configured to project from a tibial surface (56) pointing towards the meniscal component (16).

14. Knee joint endoprosthesis in accordance with any one of the preceding claims, **characterized by** a securing device (130) for securing the meniscal component (16) to the tibial component (14) in a connected position in which the meniscal component (16) and the tibial component (14) are mounted for rotation about the axis of rotation (88).

15. Knee joint endoprosthesis in accordance with any one of the preceding claims, **characterized by** a rotation delimiting stop (148) for delimiting movement of the lateral joint surface (28) in the anterior direction.

## Revendications

1. Endoprothèse d'articulation du genou (10) comprenant une pièce de fémur (12), une pièce de tibia (14) et une pièce de ménisque (16) montée entre la pièce de fémur (12) et la pièce de tibia (14), la pièce de ménisque (16) étant montée sur la pièce de tibia (14) de manière rotative autour d'un axe de rotation (88) s'étendant côté médial, **caractérisée en ce qu'**il est prévu un dispositif de guidage de rotation (104) pour forcer un mouvement de rotation de la pièce de ménisque (16) par rapport à la pièce de tibia (14) autour de l'axe de rotation (88), en réponse à un mouvement de pivotement de la pièce de fémur (12) et de la pièce de tibia (14) l'une par rapport à l'autre autour d'un axe de pivotement (106) s'étendant transversalement à l'axe de rotation (88), et **en ce que** le dispositif de guidage de rotation (104) comprend des premier et deuxième éléments de guidage (108, 110), qui sont agencés ou formés d'une part sur la pièce de fémur (12) et d'autre part sur la pièce de tibia (14).

2. Endoprothèse d'articulation du genou selon la revendication 1, **caractérisée en ce que** la pièce de fémur (12) comprend un condyle médial et un condyle latéral (18, 20), qui présentent une surface condylienne médiale et une surface condylienne latérale (22, 24), ladite pièce de ménisque (16) présentant une surface articulaire médiale et une surface articulaire latérale (26, 28), sur lesquelles s'appuient au moins partiellement les surfaces condyliennes médiale et latérale (22, 24).

3. Endoprothèse d'articulation du genou selon la revendication 2, **caractérisée en ce que** la surface condylienne médiale et/ou la surface condylienne latérale (22, 24) comportent une zone de surface condylienne (30, 32) de courbure concave, et **en ce que** la surface articulaire médiale et/ou la surface articulaire latérale (26, 28) comportent une zone de surface articulaire (34, 36) de courbure convexe correspondant à la surface condylienne médiale et/ou latérale (22, 24).

4. Endoprothèse d'articulation du genou selon la revendication 2 ou la revendication 3, **caractérisée en ce que** des rayons de courbure des surfaces articulaires médiale et/ou latérale (26, 28) sont plus grands que des rayons de courbure des surfaces condyliennes médiale et/ou latérale (22, 24).

5. Endoprothèse d'articulation du genou selon l'une des revendications précédentes, **caractérisée en ce que** l'axe de rotation (88) est défini par un palier de rotation (90) formé entre la pièce de tibia (14) et la pièce de ménisque (16).

6. Endoprothèse d'articulation du genou selon la revendication 5, **caractérisée en ce que** le palier de rotation (90) comprend des premier et deuxième éléments de palier (92, 94) interagissant mutuellement, qui sont agencés ou formés d'une part sur la pièce de tibia (14) et d'autre part sur la pièce de ménisque (16).

7. Endoprothèse d'articulation du genou selon la revendication 6, **caractérisée en ce que** l'un des éléments de palier (94) est de configuration cylindrique et l'autre élément de palier (92) est de configuration cylindrique creuse.

8. Endoprothèse d'articulation du genou selon l'une des revendications précédentes, **caractérisée en ce que** le dispositif de guidage de rotation (104) est configuré pour forcer un mouvement de roulement de la pièce de fémur (12) et de la pièce de ménisque (16) l'une contre l'autre.

9. Endoprothèse d'articulation du genou selon l'une des revendications précédentes, **caractérisée en ce que** les premier et deuxième éléments de guidage (108, 110) sont configurés pour définir un angle de rotation d'un mouvement de rotation de la pièce de ménisque (16) et de la pièce de tibia (14) autour de l'axe de rotation (88), en fonction d'un angle de flexion entre la pièce de fémur (12) et la pièce de tibia (14).

10. Endoprothèse d'articulation du genou selon l'une des revendications précédentes, **caractérisée en ce que** le premier élément de guidage (108) est réalisé dans la région antérieure de la pièce de tibia (14).

11. Endoprothèse d'articulation du genou selon l'une des revendications précédentes, **caractérisée en ce que** la pièce de ménisque (16) comprend une partie médiale de pièce de ménisque (72) et une partie latérale de pièce de ménisque (74), et **en ce que** les parties latérale et médiale de pièce de ménisque (72, 74) sont reliées mutuellement par un élément de liaison (76).

12. Endoprothèse d'articulation du genou selon la revendication 11, **caractérisée en ce que** l'élément de liaison (76) est réalisé sous la forme d'une branche (78) .

13. Endoprothèse d'articulation du genou selon l'une des revendications précédentes, **caractérisée en ce que** le premier élément de guidage (108) est réalisé de manière à être éloigné d'une surface de tibia (56) dirigée vers la pièce de ménisque (16).

14. Endoprothèse d'articulation du genou selon l'une des revendications précédentes, **caractérisée par** un dispositif d'arrêt de sécurité (130) pour sécuriser la pièce de ménisque (16) sur la pièce de tibia (14) dans une position d'assemblage, dans laquelle la pièce de ménisque (16) et la pièce de tibia (14) sont montées rotatives autour de l'axe de rotation (88).

15. Endoprothèse d'articulation du genou selon l'une des revendications précédentes, **caractérisée par** une butée de limitation de rotation (148) destinée à limiter un mouvement de la surface articulaire latérale (28) en direction antérieure.
